# EUROPEAN PATENT SPECIFICATION

(11) **EP 1 526 185 B1**
(45) Date of publication and mention of the grant of the patent: **12.09.2012**
(21) Application number: 03024283.8
(22) Date of filing: 22.10.2003
(51) Int. Cl.: C12N 15/86, A61K 48/00, A61K 39/00

(54) **Use of a microorganism or cell to induce autoimmunization of an organism against a tumor**
Verwendung von Mikroorganismen oder Zellen zur Autoimmunisierung gegen Tumoren
Utilisation des micro-organismes ou des cellules pour induire l'autoimmunisation contre des tumeurs

(43) Date of publication of application: 27.04.2005
(73) Proprietor: Genelux Corporation, San Diego CA 92109 (US)
(72) Inventor: Aladar, Szalay, Highland, CA 92346 (US)
(74) Representative: Blance, Stephen John

(56) References cited:
- WO-A-96/11279
- US-B1- 6 589 531
- KAUFMAN H ET AL: "A RECOMBINANT VACCINIA VIRUS EXPRESSING HUMAN CARCINOEMBRYONIC ANTIGEN CEA" INTERNATIONAL JOURNAL OF CANCER, vol. 48, no. 6, 1991, pages 900-907, XP009023961 ISSN: 0020-7136
- OVERWIJK W W ET AL: "Vaccination with a recombinant vaccinia virus encoding a self antigen induces autoimmune vitiligo and tumor cell destruction in mice: requirement for CD4(+) T lymphocytes" PROCEEDINGS OF THE NATIONAL ACADEMY OF SCIENCES OF USA, NATIONAL ACADEMY OF SCIENCE. WASHINGTON, US, vol. 96, no. 6, March 1999 (1999-03), pages 2982-2987, XP002226929 ISSN: 0027-8424
- DATABASE MEDLINE [Online] US NATIONAL LIBRARY OF MEDICINE (NLM), BETHESDA, MD, US January 2000 CONRY R M ET AL: 'Human autoantibodies to carcinoembryonic antigen (CEA) induced by a vaccinia-CEA vaccine.' Database accession no. NLM10656429 & CLINICAL CANCER RESEARCH : AN OFFICIAL JOURNAL OF THE AMERICAN ASSOCIATION FOR CANCER RESEARCH JAN 2000, vol. 6, no. 1, January 2000 (2000-01), pages 34-41, ISSN: 1078-0432

## Description

The present invention relates to the use of a microorganism or cell containing a DNA sequence encoding a desired polypeptide or RNA to induce autoimmunization of an organism against a tumor. Furthermore, the present invention relates to a method for the production of antibodies against a tumor comprising (a) injecting a microorganism or cell containing a DNA sequence encoding a desired polypeptide or RNA into an organism bearing a tumor and (b) isolating antibodies against the tumor.

The present applicant found that the method described in EP 03 018 478.2 relating to "The production of a polypeptide, RNA or other compound in a tumor tissue" also enables the production of antibodies against the tumor tissue. These antibodies present an autoimmunization of the organism bearing the tumor. Furthermore, these antibodies can be isolated and used for the treatment of tumors in other organisms. The term "organisms" refers to animals and human beings.

Overwijk et al. (Proc. Natl. Acad. Sci. USA, 1999, vol. 96, p. 2982-2987) discloses the recombinant vaccinia virus encoding the self antigen TRP-1, which is expressed by both normal and malignant melanocytes, induces autoimmune melanoma tumour cell destruction.

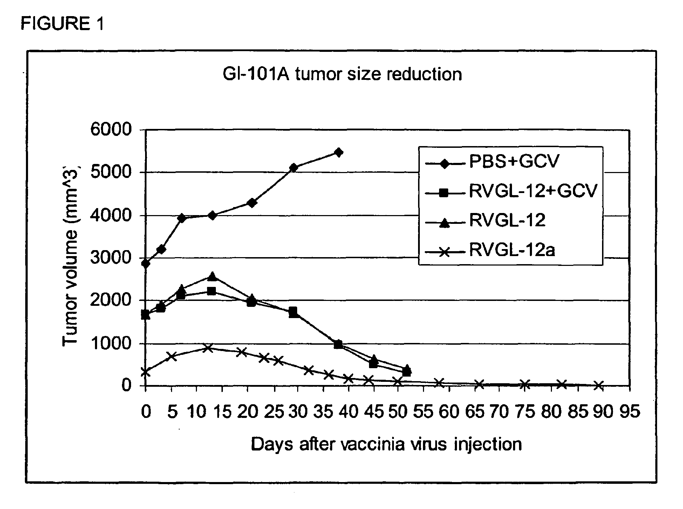

In the following the present application is exemplarily described in the reduction and elimination of xenogeneic GI-101A solid breast carcinoma tumors and their metastases in nu⁻/nu⁻ mice (T cell deficient mice).
**Step#1:** Female nu⁻/nu⁻ mice of 5 weeks age were chosen, and the GI-101A cells grown in RPMI1640 medium, supplemented with estrogen and progesterone. When reached desired confluence, cells were harvested, washed with phosphate buffered saline. Cells (5 × 10⁶ cells per mouse) were then injected subcutaneously into mice. The tumor growth was carefully monitored every two days.
**Step#2:** At two stages of tumor growth (at tumor size of 400-600 mm³, and at tumor size of ∼ 1700 mm³), purified vaccinia viral particles were delivered to each tumorous mice by intravenous injection through tail vein. The colony purified virus was amplified in CV-1 cell line and the intracellular viral particles were purified by centrifugation in sucrose gradient. Two concentrations of virus (10⁶ pfu/100 □| and 10⁷ pfu/100 □| resuspended in PBS solution) were injected. The viral replication was monitored externally by visualization of virus-mediated green fluorescence protein expression. The tumor development was monitored by tumor volume determination with a digital caliper.
**Step#3**: After viral application, it was determined that first the tumors continued to grow to a size of ∼ 900 mm³ (from 400-600 mm³ at the time of viral injection), and to a size of ∼ 2400 mm³ (from 1700 mm³). Then the growth rate leveled off for approximately 6-8 days.
**Step#4**: Approximately 14 days after viral injection, the tumor volume started to decline rapidly (Figure 1). Forty days after viral application, all the treated animals showed more than 60% tumor regression. Sixty-five days after viral treatment and many of the animals had complete regression of tumors.
**Step#5:** Some of the animals were completely tumor-free for several weeks and their body weight returned to normal.
**Step#6:** The level of immune activation was determined. According to the immunoblot analysis of the table 1 sera were obtained from the animals with regressing tumors and the immune titer determined against a foreign protein (e.g. green fluorescent protein), vaccinia viral proteins, and GI-101A cancer cell proteins.

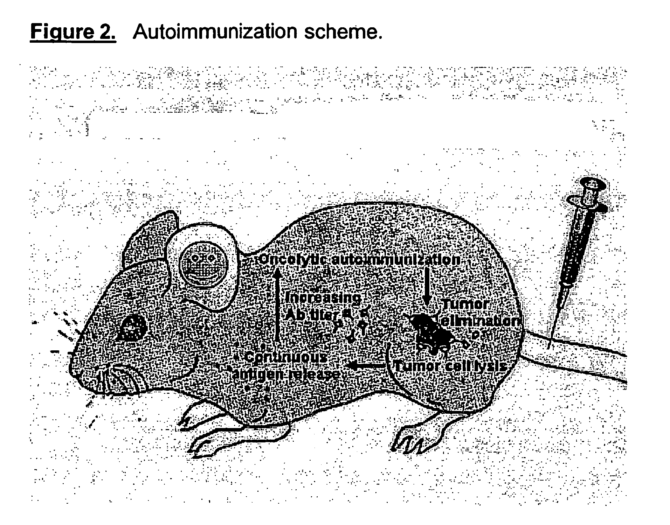

In conclusion, the present invention shows that solid tumors allowed an enormous tumor-specific vaccinia virus replication, which led to tumor protein antigen and viral protein productions in the tumors. In addition, vaccinia virus did lyse the infected tumor cells and thereby released tumor-cell-specific antigens. The continuous leakage of these antigens into the body led to a very high level of antibody titer (in approximately 7-14 days) against foreign cell proteins (tumor proteins), viral proteins, and the virus encoded engineered proteins in the mouse body. The newly synthesized antitumor antibodies and the enhanced macrophages, neutrophils counts were continuously delivered via the vasculature into the tumor and thereby provided for the recruitment of an activated immune system in the inside of the tumor. The active immune system then eliminated the foreign compounds of the tumor including the viral particles. This interconnected release of foreign antigens boosted in antibody production and continuous return of the antibodies against the tumor-contained proteins function like an autoimmunization vaccination system initiated by vaccinia viral replication, followed by cell lyses, protein leakage and enhanced antibody production (see figure 2).

Thus, the present invention teaches a complete process which may be applied to all tumor systems with immunoprivileged tumor sites as site of privileged viral, bacterial, and mammalian cell growth. These findings may lead the way for tumor elimination by the host own immune system.

### TABLE 1:

Immunoblot analysis. The antisera obtained from the following sources are used to analyze the following listed samples.
Samples:
1). Mouse cell lysate (control);
2). Purified and denatured vaccinia viral particles;
3). GI-101A tumor cell lysate;
4). Purified green fluorescent protein;
5). Purified luciferase protein;
6). Purified beta-galactosidase protein.
Antisera:
a). Antiserum from nontumorous mouse;
b). Antiserum from GI-101A tumorous mouse;
c). Antiserum from GI-101A tumorous mouse 14 days after vaccinia i.v. injection;
d). Antiserum from GI-101A tumorous mouse 65 days after vaccinia i.v. injection;
e). Antiserum from tumor-free mouse (after elimination of GI-101A tumor) 80 days after vaccinia i.v. injection.

### BRIEF DESCRIPTION OF THE DRAWINGS:

**Figure 1**. Tumor size reduction. Vaccinia viruses RVGL-12+GCV(gancyclovir), and RVGL-12 were injected on 67 days after GI-101A cellular implantation.. RVGL-12a virus was injected on 30 days after cellular implantation. RVGL-12+GCV treatment resulted in 86.3% reduction of tumor size (Day 52 after viral injection) from their peak volumes on Day 13. RVGL-12 treatment resulted in 84.5% reduction of tumor size (Day 52) from their peak volumes (Day 13). RVGL-12a treatment resulted in 98.3% reduction of tumor size (Day 89) from their peak volumes (Day 12). On the contrary, with PBS+GCV control treatment, the average volume of tumors were increased by 91.8% in 38 days.

## Claims

1. Vaccinia virus for use as an autoimmunisation vaccination system for the treatment of solid tumours wherein said autoimmunisation system is initiated by tumour-specific vaccinia virus replication leading to lysis of infected cells, continuous release of tumour cell-specific antigens and consequent antibody production against said released tumour cell-specific antigens.

2. Vaccinia virus for use as claimed in claim 1, wherein the vaccinia virus contains nucleic acid that encodes a desired polypeptide or RNA.

## Patentansprüche

1. Vaccinia-Virus zur Verwendung als Autoimmunisierungs-Impfsystem für die Behandlung fester Tumoren, wobei das Autoimmunisierungs-System durch die Replikation von Tumorspezifischem Vaccinia-Virus, welche zur Lyse infizierter Zellen führt, die fortdauernde Freisetzung von Tumorzell-spezifischen Antigenen und die daraus folgende AntikörperProduktion von Antikörpern gegen die freigesetzten Tumorzell-spezifischen Antigene ausgelöst wird.

2. Vaccinia-Virus zur Verwendung wie in Anspruch 1 beansprucht, wobei das Vaccinia-Virus Nukleinsäure enthält, die ein gewünschtes Polypeptid oder RNA kodiert.

## Revendications

1. Virus vaccinal pour une utilisation comme système de vaccination par auto-immunisation pour le traitement de tumeurs solides, ledit système d'auto-immunisation étant déclenché par la réplication du virus vaccinal spécifique de la tumeur conduisant à la lyse des cellules infectées, à la libération continue d'antigènes spécifiques des cellules tumorales et à la production résultante d'anticorps contre lesdits antigènes libérés spécifiques des cellules tumorales.

2. Virus vaccinal pour une utilisation suivant la revendication 1, ledit virus vaccinal contenant un acide nucléique qui code pour un polypeptide ou ARN désiré.
